# EUROPÄISCHE PATENTANMELDUNG

(11) **EP 0 801 073 A2**
(43) Veröffentlichungstag der Anmeldung: **15.10.1997**
(21) Anmeldenummer: 97101088.9
(22) Anmeldetag: 24.01.1997
(51) Int. Cl.: C07K 5/08, C07K 5/10, C07K 1/02

(54) **Verfahren zur Herstellung von Oligopeptid-Aldehyden**

(30) Priorität: 12.02.1996 DE 19605039
(71) Anmelder: HOECHST AKTIENGESELLSCHAFT, 65929 Frankfurt am Main (DE)
(72) Erfinder: Rybczynski, Wolfgang, Dr., 63505 Langenselbold (DE); Kesseler, Kurt, Dr., 65835 Liederbach (DE)

(57) **Zusammenfassung**

Die Erfindung betrifft ein Verfahren zur Herstellung von Oligopeptid-Aldehyden, welches dadurch gekennzeichnet ist, daß ein am N-terminalen Ende reversibel geschützter Oligopeptid-Alkohol in einem gepufferten Lösungsmittelgemisch, enthaltend ein nicht oxidierbares, polares, nicht mit Wasser mischbares Lösungsmittel und ein wenig Wasser gelöst wird, mit einer stöchiometrischen Menge an Natriumbromid versetzt wird, der Alkohol unter Kühlung mit einer katalytischen Menge an 2,2,6,6-Tetramethyl-piperidin-N-oxyl (TEMPO) oder eines Derivates hiervon oxidiert wird, die Reaktions-Suspension mit einer wäßrigen Natriumhypochlorit-Lösung versetzt wird und schließlich die Reaktion durch Zugabe eines Reduktionsmittels beendet wird.

Die so hergestellten Oligopeptid-Aldehyde sind in Detergenzien zu verwenden.

## Beschreibung

Die Erfindung betrifft ein Verfahren zur Herstellung von Oligopeptid-Aldehyden, die in Detergenzien verwendet werden können.

Oligopeptid-Aldehyde und ihre Verwendung in flüssigen Detergenzien sind allgemein bekannt. So beschreibt die WO 94/04651, daß Peptid-Aldehyde vorteilhaft in geringen Konzentrationen eingesetzt werden können, um dennoch in hohem Maße als sensible Inhibitoren der Protease zu wirken. Das bietet den zusätzliche Vorteil, daß nun in Detergenzien auch Enzyme zugesetzt werden können, die ihrerseits empfindlich gegenüber einem proteolytischen Abbau sind.

Die Herstellung der Oligopeptid-Aldehyde kann gemäß des US-A-4,399,065 (DE-A-32 00 812) erfolgen, hier am Beispiel eines Tripeptids im maßgebenden Schritt ausgehend von einem Lactam, das mit Lithiumaluminiumhydrid in THF zum Aldehyd reduziert wird. Dieses Verfahren wird auch in der EP-A-0 185 390 angewendet.

Gemäß der WO 94/04651 erfolgt die Herstellung eines Tetrapeptid-Aldehyds in zwei Stufen. Zuerst wird aus dem Ester eines Tetrapeptids mittels NaBH₄ in Ethanol und THF der entsprechende Alkohol erhalten, der seinerseits anschließend unter Einwirkung von "Dess-Martin" in CH₂Cl₂ nach Weiterreaktion in NaHCO₃/Na₂S₂O₃ zum Aldehyd aufoxidiert wird.

Das zuletzt genannte Verfahren besitzt Nachteile, die es zu verbessern galt. Grundsätzlich hat das Zweistufen-Verfahren eine geringere Raum/Zeit-Ausbeute als ein mögliches einstufiges Verfahren. Darüber hinaus Zeigen die verwendeten Chemikalien ihrerseits Nachteile, einmal durch sie selbst als auch in ihrer Wirkung im Verfahrensablauf: Verfahren, die auf die Verwendung von CH₂Cl₂ angewiesen sind, sind von vornherein nicht vorteilhaft. Die Verwendung von NaBH₄ und "Dess-Martin" schließen aufgrund von Sicherheitsaspekten von vornherein eine Übertragung einer solchen Reaktion auf technische Maßstäbe aus (geringe Verfügbarkeit, Preis, exotherme Reaktion, Sicherheit der Reaktionsführung). Weiterhin ist die Verwendung von NaHCO₃ aufgrund der geringen Löslichkeit in Wasser nachteilig. Die genannten Reaktionsschritte (Reduktion/Oxidation) wurden unter Verwendung der Schutzgruppe BOC am Amino-terminalen Ende des Oligopeptids durchgeführt. Hier wäre es wünschenswert, Bedingungen zu schaffen, die eine leichtere Freisetzung der Schutzgruppe ermöglichten.

Aufgabe der Erfindung war es deshalb, ein neues Verfahren zu entwickeln, indem ein Oligopeptid am C-terminalen Ende in einen Oligopeptid-Aldehyden übergeführt wird. Das Verfahren sollte die genannten Nachteile des oben beschriebenen Standes der Technik nicht mehr aufweisen. Das erfindungsgemäße Verfahren sollte geeignet sein, auch in technischem Maßstab eingesetzt werden zu können.

Die Lösung der Aufgabe wird erreicht durch ein Verfahren zur Herstellung eines reversibel geschützten Oligopeptid-Ahydehyds, dadurch gekennzeichnet, daß ein am N-terminalen Ende reversibel geschützter Oligopeptid-Alkohol in einem gepufferten Lösungsmittelgemisch, enthaltend ein nicht oxidierbares, polares, nicht mit Wasser mischbares Lösungsmittel und wenig Wasser gelöst wird, mit einer stöchiometrischen Menge an Natriumbromid versetzt wird, der Alkohol unter Kühlung mit einer katalytischen Menge an 2,2,6,6-Tetramethyl-piperidin-N-oxyl (TEMPO) oder eines Derivates hiervon oxidiert wird, die Reaktions-Suspension mit einer wäßrigen Natriumhypochlorit-Lösung versetzt wird und schließlich die Reaktion durch Zugabe eines Reduktionsmittels beendet wird.

Die Reaktionsmischung wird aufgearbeitet, indem die beiden Phasen getrennt werden und die wäßrige Phase mit einem nicht oxidierbaren, polaren und nicht mit Wasser mischbaren Lösungsmittel extrahiert wird und schließlich aus den vereinigten organischen Phasen der N-terminal geschützte Oligopeptid-Aldehyd erhalten wird.

Als nicht-oxidierbares, polares, nicht mit Wasser mischbares Lösungsmittel kommt insbesondere Ethylacetat in Frage.

Der Gehalt an Wasser im Lösungsmittelgemisch muß erfindungsgemäß so gering wie möglich gehalten werden, insbesondere ≤10 %, bevorzugt ≤5 %.

Als Puffer für das Lösungsmittelgemisch werden insbesondere Natriumhydrogencarbonat oder Kaliumhydrogencarbonat verwendet. Besonders bevorzugt ist Natriumhydrogencarbonat als 1 - 10 %ige, bevorzugt 3 - 5 %ige Lösung.

Als Katalysator für die Oxidationsreaktion zum Aldehyd wird 2,2,6,6-Tetramethylpiperidin-N-oxyl (TEMPO) oder eines seiner Derivate eingesetzt. Als Derivate kommen das 4-Hydroxy-Derivat oder das eher zu empfehlende 4-Acetamid-Derivat in Frage. Besonders bevorzugt ist allerdings das unsubstituierte TEMPO (nicht derivatisiert).

Die Natriumhypochlorit-Lösung ist 10 - 20 %ig, bevorzugt 12 - 15 %ig.

Vor der Phasentrennung wird die Reaktion erfindungsgemäß in der Suspension durch Zugabe eines Reduktionsmittels beendet. Als Reduktionsmittel werden solche bevorzugt, die in Wasser löslich sind. Besonders bevorzugt sind Dithionite, Sulfite und/oder Thiosulfate. Insbesondere bevorzugt ist Alkalithiosulfat, besonders Natriumthiosulfat-(Pentahydrat).

Als Schutzgruppen am N-terminalen Ende können alle dem Fachmann bekannten Schutzgruppen, die wieder abspaltbar sind (reversibel) verwendet werden. Zu nennen sind beispielsweise Benzyloxycarbonyl, tert.-Butyloxycarbonyl (BOC), Methyloxycarbonyl (MOC), Ethyloxycarbonyl (EtOC). Besonders bevorzugt sind MOC und/oder EtOC.

Die sich bildenden zwei Phasen der Reaktionsmischung (Suspension) werden getrennt und die wäßrige Phase hiervon, insbesondere mit Ethylacetat extrahiert. Die vereinigten organischen Phasen werden mit Wasser nachextrahiert.

Die Produktisolation erfolgt durch Abdestillation des Wasser/Ethylacetat-Gemisches. Eine zusätzliche Reinigung kann durch ein anschließendes Fällen des Produktes (des Aldehydes) aus einem unpolaren Lösungsmittel, insbesondere aus n-Propan oder n-Hexan, erreicht werden.

Nach Zugabe des Natriumbromids muß das Reaktionsgemisch auf -5 bis +5°C, insbesondere auf Temperaturen um 0°C gekühlt werden und solange auf dieser Temperatur gehalten werden, bis die Reaktion durch Zusatz des Reduktionsmittels beendet wird.

Nach dem erfindungsgemäßen Verfahren sind die Oligopeptid-Aldehyde in überraschend hohen Ausbeuten von 90 - 100 %, vorzugsweise 90 - 96 % herstellbar.

Durch das erfindungsgemäße Verfahren wird vorteilhaft die Entstehung von Nebenprodukten sehr gesenkt. Eine Überoxidation des Aldehyds ist fast vollständig unterdrückt.

TEMPO als Oxidationskatalysator ist aus Tetrahedron Letters, Vol. 33, No. 35, Seiten 5029-5032 (1992) nur für die Oxidation von α-Amino-Alkoholen und α-Alkoxy-Alkoholen zu den entsprechenden Aldehyden bekannt. Dort wird die Reaktion in einen Gemisch aus Toluol/Wasser und unter Verwendung eines Natriumhydrogencarbonatpuffers durchgeführt. Die Zugabe des Reduktionsmittels erfolgt hier nach Trennung der Phasen in die organische Phase. Diese Reaktionsführung läßt aber nur geringe Ausbeuten zu. Insbesondere kann man die Überoxidation des Reaktionsproduktes nicht hinreichend genug regeln. Die beschriebene schnelle Durchmischung des Reaktionsgemisches scheint auf die Überoxidation keinen großen Einfluß zu haben (Seite 5030, 2. Absatz, 2. und 3. Satz).

Einer Übertragung der Oxidationsbedingungen für die Oxidation der niedermolekularen α-Amino-Alkohole und α-Alkoxy-Alkohole mit TEMPO auf die Verhältnisse bei den höhermolekularen und damit unpolareren Oligopeptid-Alkoholen steht insbesondere entgegen, daß gemäß den Beispielen dieser Literaturstelle, die Ausbeuten umso geringer werden, je längerkettiger das Molekül ist und zusätzlich der N-Terminus nur einfach geschützt ist. Der Fachmann würde daher diese Methode generell nicht für die Oxidation von längerkettigen, mit nur einfach geschützten N-terminalen Oligopeptiden in Betracht ziehen. Bei einer Übertragung hätte man daher mit noch geringeren Ausbeuten zu rechnen, als sie schon bei den niedermolekularen Verbindungen anzutreffen sind. Umso überraschender war es, daß durch die erfindungsgemäße Verfahrensweise gerade mit dem Oxidationskatalysator TEMPO unter den erfindungsgemäßen Bedingungen sehr hohe Ausbeuten zu erzielen sind.

Gegenüber dem genannten Verfahren der Oxidation von α-Alkoxy-Alkoholen und α-Amino-Alkoholen mit TEMPO hat das erfindungsgemäße Verfahren neben der höheren Ausbeute auch noch folgende überraschende Vorteile:

Nach der Zugabe des Reduktionsmittels, hier Natriumthiosulfat, kann auf die Zugabe von Phosphat zur Einstellung eines pH-Wertes von 7 verzichtet werden.

Ebenso wird eine zusätzliche Extraktion mit gesättigter NaCl-Lösung eingespart. Schließlich wird nur die Herstellung eines Rohproduktes beschrieben, nicht aber, wie man ein Oligopeptid in Reinform erhält. Die vorteilhafte Fällung im erfindungsgemäßen Verfahren aus einem unpolaren Lösungsmittel, wie z.B. n-Heptan oder n-Hexan, führt zu hochreinem Produkt.

Unter Oligopeptid-Aldehyden zur Anwendung im Sinne des erfindungsgemäßen Verfahrens werden Peptid-Aldehyde mit 2 bis 50 Aminosäuren oder Mischungen derselben verstanden. Als Ausgangspunkt für das erfindungsgemäße Verfahren werden Peptid-Ketten eingesetzt, deren C-terminale Enden Alkoholfunktionen (-OH) aufweisen, die im Verfahren selbst in die entsprechenden Aldehyd-Funktionen (-H) übergeführt werden. Die Oligopeptid-Aldehyde sind als solche und die Verfahren ihrer Herstellung aus US-A-5 015 627, EP-A-0 185 930 und DE-A-32 00 812 bekannt.

Für das erfindungsgemäße Verfahren sind Oligopeptid-Aldehyde bevorzugt, die aus 2 bis 10, bevorzugt aus 2 bis 6 und besonders bevorzugt aus 3 oder 4 Aminosäuren bestehen.

Zu nennen sind, je nach Anwendungsgebiet und vorliegender Protease im Detergenz, folgende Oligopeptid-Aldehyde mit 3 Aminosäuren:
Lys-Ala-LysH, Ile-Phe-LysH, Phe-Pro-ArgH, Phe-Val-ArgH, Lys-Ala-AlaH, Ala-Ala-ProH, Gly-Ala-LeuH, Gly-Ala-LeuH, Gly-Ala-PheH, Leu-Leu-PheH, Ala-Ala-PheH, Leu-Leu-TyrH, Val-Pro-ValH, Ala-Val-LeuH, Lys-Ala-AlaH, Ala-Ala-ProH, Gly-Ala-LeuH und Gly-Ala-PheH.

Weiterhin sind Oligopeptid-Aldehyde mit 4 Aminosäuren zu nennen:
Phe-Gly-Ala-PheH, Phe-Gly-Ala-LeuH.

Besonders bevorzugt sind die Oligopeptid-Aldehyde mit 4 Aminosäuren, insbesondere aus Phe, Gly, Ala und Leu. Insbesondere bevorzugt ist Phe-Gly-Ala-LeuH.

Das N-terminale Ende des Oligopeptid-Aldehyds ist im erfindungsgemäßen Verfahren durch eine dem Fachmann bekannte Schutzgruppe geschützt. Bevorzugt sind die Schutzgruppen BOC und EtOC, besonders bevorzugt ist EtOC. (BOC = Butyloxycarbonyl; EtOC = Ethyloxycarbonyl).

Die Herstellung der für das erfindungsgemäße Verfahren notwendigen Oligopeptid-Alkohole sind dem Fachmann bekannt. Zu nennen ist hierbei die Patentliteratur (US-A-5 015 627, EP-A-185 930, DE-A-32 00812) aber auch die Lehrbücher: Houben-Weyl: Methoden der organischen Chemie, Stuttgart 1994, Band 15/1 und Band 15/2, Ullmanns Encyclopädie der technischen Chemie, Weinheim 1980, 4. Auflage, Band 19, Seiten 542-551 sowie andere Publikationen, wie beispielsweise: J. Johnes: Amino Acid and Peptid Synthesis, Oxford Science Publications, Oxford 1992.

Die Zielverbindung, der N-terminal geschützte Oligopeptid-Alkohol, ist über die klassische Kupplung in Lösung herzustellen. Zum einen kann dies durch eine serielle Synthese, d.h. den schrittweisen Peptidaufbau durch Ankopplung einzelner Aminosäurebausteine erfolgen; zum anderen durch eine konvergente Synthese, in der durch schrittweisen Peptidaufbau zwei, im bevorzugten Fall Dipeptide, aufgebaut werden, die in einem anschließenden Schritt (Konvergenz) miteinander gekoppelt werden.

Bei der Synthese können wiederum die an sich bekannten Schutzgruppen für die N-Terminalen und C-Terminalen eingesetzt werden.
N-Terminale: Benzyloxycarbonyl, tert.-Butyloxycarbonyl, Methyloxycarbonyl, Ethyloxycarbonyl.
C-Terminale: Methylester, Ethylester, Benzylester, Nitrobenzylester.

Die eigentliche Verknüpfung der Peptidbausteine (Peptidbinding) ist dem Fachmann auch bekannt. Hier sollen - zur Verdeutlichung - folgende Methoden genannt werden:
- Kupplung über das gemischte Anhydrid, das sich z.B. durch Umsetzung mit Chlorameisensäure-isobutylester oder Propanphosphonsäureanhydrid herstellen läßt,
- Kupplung über den O-acylierten Isoharnstoff, der durch die Umsetzung mit Dicyclohexylcarbodiimid entsteht (DCC-Methode),
- Kupplung über einen Aktivester oder die
- Kupplung über das Carbonsäureazid.

Das folgende Beispiel soll das erfindungsgemäße Verfahren zur Herstellung eines Oligopeptid-Aldehyds, hier das EtOC-Phe-Gly-Ala-Leu-H, verdeutlichen.

3,8 g EtOC-Phe-Gly-Ala-Leucinol (Leu-OH) wurden unter Rühren in 100 ml Ethylacetat gelöst, und das Gemisch nach Zugabe von 10 ml Wasser und 0,9 g Natriumbromid auf 0°C abgekühlt. Anschließend wurden 4,8 g Kaliumhydrogencarbonat, sowie 0,01 g TEMPO (2,2,6,6-Tetramethyl-piperidin-N-oxyl) zugegeben. Zu der resultierenden Suspension wurde unter Kühlung und intensivem Rühren innerhalb 15 min ein Gemisch aus 5 g Natriumhypochlorit (13 %ig) und 10 ml Wasser zugetropft. Nachdem weitere 10 min bei 0°C weitergerührt wurden, wurde die Suspension durch Zusatz einer Lösung von 3 g Natriumthiosulfat Pentahydrat in 27 ml Wasser abgeschreckt.

Die beiden Phasen des Reaktionsgemisches wurden voneinander getrennt; die wäßrige Phase wurde mit insgesamt 20 ml Ethylacetat extrahiert. Die vereinigten organischen Phasen Wurden schließlich mit Wasser nachextrahiert. Nachdem das Lösungsmittel im Vakuum eingeengt worden war, konnten nach Fällung mit n-Heptan 3,7 g reines Aldehyd gewonnen werden (96 % d.Th.)

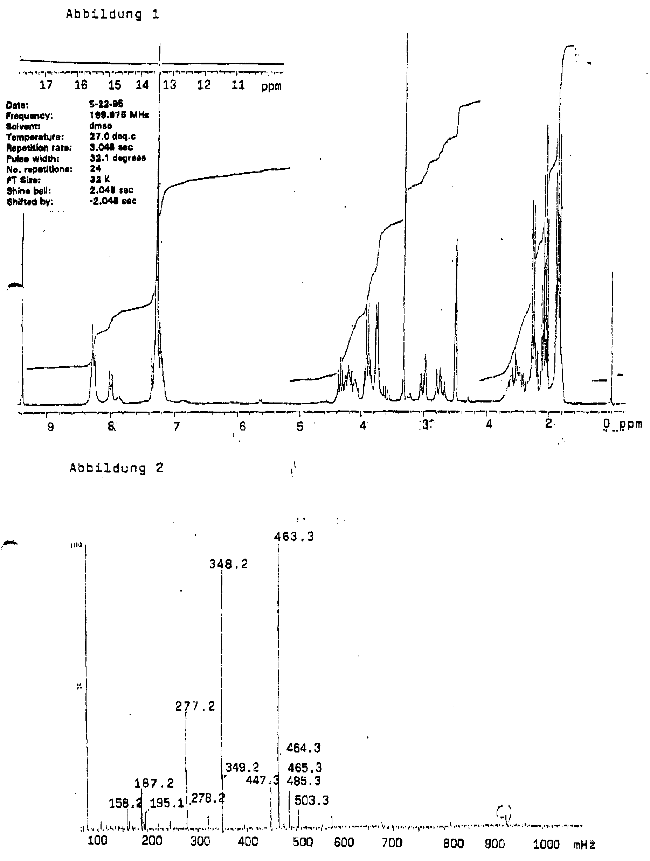

Das Produkt wurde anhand des in den Abbildungen 1 und 2 dargestellten ¹H-NMR- und MS-Spektrums als EtOC-Phe-Gly-Ala-Leu-H charakterisiert.

## Patentansprüche

1. Verfahren zur Herstellung eines reversibel geschützten Oligopeptid-Aldehyds, dadurch gekennzeichnet, daß ein am N-terminalen Ende reversibel geschützter Oligopeptid-Alkohol in einem gepufferten Lösungsmittelgemisch, enthaltend ein nicht oxidierbares, polares, nicht mit Wasser mischbares Lösungsmittel und wenig Wasser gelöst wird, mit einer stöchiometrischen Menge an Natriumbromid versetzt wird, der Alkohol unter Kühlung mit einer katalytischen Menge an 2,2,6,6-Tetramethyl-piperidin-N-oxyl (TEMPO) und/oder eines Derivates hiervon oxidiert wird, die Reaktions-Suspension mit einer wäßrigen Natriumhypochlorit-Lösung versetzt wird und schließlich die Reaktion durch Zugabe eines Reduktionsmittels beendet wird.

2. Verfahren nach Anspruch 1, dadurch gekennzeichnet, daß als Schutzgruppe am N-terminalen Ende Benzyloxycarbonyl, tert. Butyloxycarbonyl, Methyloxycarbonyl oder Ethyloxycarbonyl, verwendet werden.

3. Verfahren nach den Ansprüchen 1 oder 2, dadurch gekennzeichnet, daß als nicht-oxidierbares, polares, nicht mit Wasser mischbares Lösungsmittel Ethylacetat im Lösungsmittelgemisch eingesetzt wird.

4. Verfahren nach den Ansprüchen 1 oder 3, dadurch gekennzeichnet, daß das Lösungsmittelgemisch einen Wassergehalt von bis zu 10 %, bevorzugt bis zu 5 % aufweist.

5. Verfahren nach den Ansprüchen 1, 3 oder 4, dadurch gekennzeichnet, daß als Puffer für das Lösungsmittelgemisch Natrium- und/oder Kaliumhydrogencarbonat, vorzugsweise Kaliumhydrogencarbonat verwendet werden.

6. Verfahren nach Anspruch 1, dadurch gekennzeichnet, daß als Katalysator TEMPO und/oder ein Derivat hiervon, beispielsweise das 4-Hydroxy- oder das 4-Acetamid-Derivat verwendet werden.

7. Verfahren nach den Ansprüchen 1 bis 6, dadurch gekennzeichnet, daß als Reduktionsmittel Dithionite, Sulfite und/oder Thiosulfate, insbesondere Natriumthiosulfat, verwendet werden.

8. Verfahren nach den Ansprüchen 1 bis 7, dadurch gekennzeichnet, daß als Oligopeptid-Alkohole solche mit 2 bis 10, bevorzugt mit 2 bis 6 und insbesondere bevorzugt mit 3 oder 4 Aminosäuren, eingesetzt werden.

9. Verfahren nach den Ansprüchen 1 und 8, dadurch gekennzeichnet, daß als Oligopeptid-Alkohole solche mit den Aminosäuren Phe, Gly, Ala und Leu oder Phe, Gly, Ala und Pe verwendet werden, insbesondere Phe-Gly-Ala-LeuOH.

10. Verfahren nach den Ansprüchen 1 bis 9, dadurch gekennzeichnet, daß das Oligopeptid-Aldehyd durch Kristallisation aus n-Heptan und/oder n-Hexan erhältlich ist.

11. Verfahren nach den Ansprüchen 1 bis 10, dadurch gekennzeichnet, daß der Oligopeptid-Alkohol in einer seriellen Synthese oder einer konvergenten Synthese, bevorzugt in einer konvergenten Synthese herstellbar ist.

12. Oligopeptid-Aldehyde, herstellbar nach einem Verfahren eines oder mehrerer Ansprüche 1 bis 11.


SEQUENZPROTOKOLL

(1) ALLGEMEINE INFORMATION:

(i) ANMELDER:
(A) NAME: Hoechst Aktiengesellschaft
(B) STRASSE: -
(C) ORT: Frankfurt
(D) BUNDESLAND: -
(E) LAND: Germany
(F) POSTLEITZAHL: 65926
(G) TELEPHON: 069-305-7975
(H) TELEFAX: 069-35-7175

(ii) ANMELDETITEL: Verfahren zur Herstellung von
Oligopeptid-Aldehyden

(iii) ANZAHL DER SEQUENZEN: 2

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 4 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..4
(D) Sonstige Angaben:\note = die Aminosäure in Lage 4 enthält
anstelle einer Carboxylgruppe eine Aldehydgruppe

(xi) SEQUENZBESCHREIBUNG: SEQ ID NO: 1:

Phe Gly Ala Phe
1

(2) INFORMATION ZU SEQ ID NO: 2:

(i) SEQUENZ CHARAKTERISTIKA:
(A) LÄNGE: 4 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..4
(D) Sonstige Angaben:\note = die Aminosäure in Lage 4 enthält
anstelle einer Carboxylgruppe eine Aldehydgruppe

(xi) SEQUENZBESCHREIBUNG: SEQ ID NO: 2:

Phe Gly Ala Leu
1